# FASCICULE DE BREVET EUROPEEN

(11) **EP 0 514 491 B1**
(45) Date de publication et mention de la délivrance du brevet: **03.11.1993**
(21) Numéro de dépôt: 91905300.9
(22) Date de dépôt: 13.02.1991
(51) Int. Cl.: A61K 7/42

(54) **COMPOSITION COSMETIQUE FILTRANTE PHOTOSTABLE CONTENANT UN FILTRE UV-A ET UN BETA, BETA-DIPHENYLACRYLATE OU ALPHA-CYANO-BETA, BETA-DIPHENYLACRYLATE D'ALKYLE**
LICHTBESTÄNDIGE FILTERNDE KOSMETISCHE ZUSAMMENSETZUNG ENTHALTEND EINEN UVA-FILTER UND EIN BETA-BETA-DIPHENYLACRYLAT ODER EINEN ALPHA-CYANO-BETA-BETA-DIPHENYLACRYLSÄURE-ALKYLESTER
LIGHT-RESISTANT FILTERING COSMETIC COMPOSITION CONTAINING A UV-A FILTER AND AN ALKYL BETA, BETA-DIPHENYLACRYLATE OR AN ALKYL ALPHA-CYANO-BETA, BETA-DIPHENYLACRYLATE

(30) Priorité: 14.02.1990 FR 9001761
(43) Date de publication de la demande: 25.11.1992
(73) Titulaire: L'OREAL, 75008 Paris (FR)
(72) Inventeur: DEFLANDRE, André, F-60560 Orry-la-Ville (FR); DUBOIS, Michel, F-77120 Coulommiers (FR); FORESTIER, Serge, F-77410 Claye-Souilly (FR); RICHARD, Hervé, F-75020 Paris (FR)
(74) Mandataire: Casalonga, Axel
(86) Numéro de dépôt international: FR9100112
(87) Numéro de publication internationale: WO9111989

(56) Documents cités:
- FR-A- 1 368 808
- FR-A- 2 440 933
- US-A- 4 810 489

## Description

La présente invention est relative à une composition cosmétique photostable destinée à protéger la peau du rayonnement UV, contenant en association un filtre UV-A et un β,β-diphénylacrylate ou α-cyano-β,β-diphénylacrylate d'alkyle, à son utilisation pour la protection de la peau contre les rayons UV et à un procédé de stabilisation du filtre UV-A par un β,β-diphénylacrylate ou α-cyano-β,β-diphénylacrylate d'alkyle.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc aussi souhaitable de filtrer le rayonnement UV-A.

Les brevets français n° 2 326 405 et n° 2 440 933 et le brevet européen n° 114607 décrivent des dérivés du dibenzoylméthane à titre de filtres UV-A. Il est proposé dans ces brevets d'associer ces filtres UV-A à différents filtres UV-B dans le but d'absorber l'ensemble du rayonnement UV de longueurs d'onde comprises entre 280 et 380 nm.

Malheureusement, lorsqu'ils sont utilisés en association avec ces filtres UV-B, les dérivés de dibenzoylméthane décrits dans les brevets mentionnés ci-dessus, ne possède pas toujours une stabilité photochimique suffisante pour garantir une protection constante durant une exposition solaire prolongée, ce qui nécessite des applications répétées à intervalles réguliers et rapprochés si l'on veut obtenir une protection efficace de la peau contre les rayons UV.

La demanderesse a découvert qu'en associant les dérivés de dibenzoylméthane mentionnés ci-dessus à un β,β-diphénylacrylate ou α-cyano-β,β-diphénylacrylate d'alkyle de formule 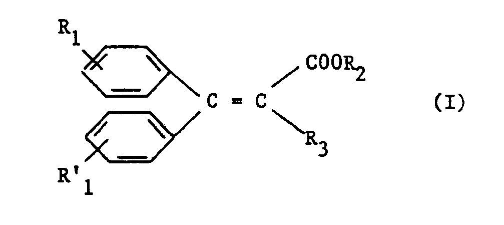
dans des proportions et dans un rapport molaire bien définis, on obtenait, de manière surprenante, une stabilité photochimique remarquable des dérivés de dibenzoylméthane. De plus, une telle association confère à la composition filtrante la contenant un fort indice de protection solaire ainsi qu'une bonne rémanence; on peut définir la rémanence comme étant la faible variation de l'indice de protection après que le sujet, ayant reçu une application de la composition filtrante, se soit baigné.

Dans la formule générale (I), les substituants R₁ à R₃ peuvent prendre les significations suivantes :
- R₁ et R'₁, identiques ou différents, représentent un atome d'hydrogène, un radical alcoxy en C₁-C₈ à chaîne droite ou ramifiée ou un radical alkyle en C₁-C₄ à chaîne droite ou ramifiée,
   R₁ et R'₁ étant en position para ou méta;
- R₂ représente un radical alkyle en C₁-C₁₂ à chaîne droite ou ramifiée;
- R₃ représente un atome d'hydrogène ou le radical CN.

Parmi les radicaux alcoxy en C₁-C₈ à chaîne droite ou ramifiée, on peut citer par exemple les radicaux méthoxy, éthoxy, n-propoxy, isopropoxy, n-butoxy, isobutoxy, tert.-butoxy, n-amyloxy, isoamyloxy, néopentyloxy, n-hexyloxy, n-heptyloxy, n-octyloxy et 2-éthylhexyloxy.

Parmi les radicaux alkyle en C₁-C₄ à chaîne droite ou ramifiée, on peut citer plus particulièrement les radicaux méthyle, éthyle, n-propyle, isopropyle, n-butyle, isobutyle, tert.-butyle. Pour les radicaux alkyle en C₁-C₁₂, on peut citer à titre d'exemple, en plus de ceux mentionnés ci-dessus, les radicaux n-amyle, isoamyle, néopentyle, n-hexyle, n-heptyle, n-octyle, 2-éthylhexyle, décyle et lauryle.

Parmi les composés de formule générale (I), on préfère plus particulièrement les composés suivants :
1 - l'α-cyano-β,β-diphénylacrylate de 2-éthylhexyle,
2 - l'α-cyano-β,β-diphénylacrylate d'éthyle,
3 - le β,β-diphénylacrylate de 2-éthylhexyle
4 - le β,β-di(4'-méthoxyphényl)acrylate d'éthyle.

Parmi les dérivés de dibenzoylméthane mentionnés dans les brevets ci-dessus, on peut citer en particulier :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4' -diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane.
- le 2,6-diméthyl-4-tert.-butyl-4'-méthoxy-dibenzoylméthane.

Parmi les dérivés de dibenzoylméthane mentionnés ci-dessus, on préfère en particulier le 4-tert.-butyl-4'-méthoxybenzoylméthane vendu sous la dénomination "PARSOL 1789" par la Société GIVAUDAN et le 4-isopropyl-dibenzoylméthane vendu sous la dénomination "EUSOLEX 8020" par la Société MERCK.

Les dérivés de formule (I) dans laquelle R₃ = CN, R₁, R'₁ et R₂ ayant les significations indiquées ci-dessus, peuvent être préparés selon le procédé décrit dans le brevet US 3 215 724.

Les dérivés de formule (I) dans laquelle R₃ = H, R₁, R'₁ et R₂ ayant les significations indiquées ci-dessus, peuvent être préparés selon le procédé décrit par L.H. KLEMM et al. dans J. Org. Chem., 23 (1958) page 344. Ce procédé permet de préparer les esters éthyliques (R₂ = C₂H₅). Pour préparer les autres esters (R₂ ≠ C₂R₅), on effectue une transestérification de l'ester éthylique par un alcanol R₂OH (R₂ ayant la signification indiquée ci-dessus sauf éthyle), en présence d'acide paratoluène sulfonique.

De par leur caractère lipophile, les filtres utilisés se répartissent uniformément dans les supports cosmétiques classiques contenant au moins une phase grasse et peuvent ainsi être appliqués sur la peau pour constituer un film protecteur efficace.

La présente invention a donc pour objet une composition cosméti- que photostable, protégeant la peau contre le rayonnement UV de longueurs d'onde comprise entre 280 et 380 nm, comprenant dans un support cosmétiquement acceptable contenant au moins une phase grasse, 1 à 5% en poids de dérivé de dibenzoylméthane et au moins 1% en poids de composé de formule (I), le rapport en moles du composé de formule (I) au dérivé de dibenzoylméthane étant supérieur ou égal à 0,8, et de préférence supérieur ou égal à 1,2.

Pour des raison de solubilisation de composés dans le composition, ce rapport est de préférence inférieur ou égal à 8, mais cette limite supérieure n'est pas critique.

La présente invention a aussi pour objet un procédé de traitement cosmétique de l'épiderme humain en vue de sa protection contre les rayons UV de longueurs d'onde comprises entre 280 et 380 nm consistant à appliquer sur la peau une quantité efficace d'une composition cosmétique telle que définie ci-dessus.

Un autre objet de la présente invention est constitué par un procédé de stabilisation des dérivés de dibenzoylméthane vis-à-vis du rayonnement UV à l'aide d'un composé de formule (I), procédé dans lequel on utilise au moins 1% en poids de composé de formule (I) pour stabiliser 1 à 5% en poids de dérivé de dibenzoylméthane, le rapport en moles du composé de formule (I) au dérivé de dibenzoylméthane étant égal ou supérieur à 0,8.

En raison du caractère lipophile des composés de formule (I) et des dérivés de dibenzoylméthane, les compositions cosmétiques selon l'invention contiennent au moins une phase grasse. Elles peuvent se présenter sous forme de lotions huileuses ou oléoalcooliques, sous forme de gels gras ou oléoalcooliques, de bâtonnets solides, d'émulsions telles qu'une crème ou un lait, ou de dispersions vésiculaires de lipides amphiphiles ioniques ou non-ioniques; elles peuvent être conditionnées en aérosol.

Comme solvant de solubilisation, on peut utiliser une huile ou une cire, un monoalcool ou un polyol inférieur ou leurs mélanges. Les monoalcools ou polyols particulièrement préférés sont l'éthanol, l'isopropanol, le propylène glycol et la glycérine.

La composition cosmétique selon l'invention destinée à protéger l'épiderme humain contre les rayons ultraviolets peut contenir les adjuvants cosmétiques usuels dans ce type de composition tels que des épaississants, des adoucissants, des humectants, des tensio-actifs, des conservateurs, des anti-mousses, des huiles, des cires, de la lanoline, des parfums, des propulseurs, des colorants et/ou des pigments ayant pour fonction de colorer la composition elle-même ou la peau, ou tout autre ingrédient habituellement utilisé en cosmétique.

En plus du composé de formule (I) et du dérivé de dibenzoylméthane, la composition peut contenir d'autres filtres UV lipophiles.

Une forme de réalisation de l'invention est une émulsion sous forme de crème ou de lait comprenant, en plus du composé de formule (I) associé au dérivé de dibenzoylméthane, des alcools gras, des esters d'acides gras et notamment des triglycérides d'acides gras, des acides gras, de la lanoline, des huiles ou des cires naturelles ou synthétiques et des émulsionnants, en présence d'eau.

Une autre forme de réalisation est constituée de lotions huileuses à base d'esters d'acides gras, d'huiles et/ou de cires naturelles ou synthétiques ou de lotions oléoalcooliques à base d'huiles, de cires ou d'esters d'acides gras tels que les triglycérides d'acides gras et d'alcools inférieurs tels que l'éthanol ou de glycols tels que le propylène glycol et/ou de polyols comme la glycérine.

Les gels oléoalcooliques comprennent une huile ou une cire, un alcool ou un polyol inférieur comme l'éthanol, le propylène glycol ou la glycérine et un épaississant tel que la silice.

Les bâtonnets solides sont constitués de corps gras comme les cires et huiles naturelles ou synthétiques, les alcools gras, les esters d'acides gras, et la lanoline.

Dans le cas d'une composition conditionnée en aérosol, on utilise les propulseurs classiques tels que les alcanes, les fluoroalcanes et les chlorofluoroalcanes.

Lorsque la composition se présente sous forme d'émulsion ou de dispersion vésiculaire, la phase aqueuse peut contenir des filtres UV hydrosolubles tels que l'acide benzène 1,4-[di(3-méthylidène 10-campho sulfonique)], l'acide 2-phénylbenzimidazole 5-sulfonique ou l'acide 2-hydroxy-4-méthoxybenzophénone 5 sulfonique (UVINUL MS 40), ces acides étant salifiés ou non.

Les exemples suivants sont destinés à illustrer l'invention.

### EXEMPLE 1

### Emulsion non ionique

- α-cyano-β,β-diphénylacrylate de 2-éthylhexyle (UVINUL N 539) 7,5 g
- 4-tert.-butyl-4'-méthoxy dibenzoylméthane (PARSOL 1789) 1,5 g
- Mélange d'alcool cétylstéarylique et d'alcool cétylstéarylique oxyéthyléné à 33 moles d'oxyde d'éthylène 7,0 g
- Mélange de mono-, di- et tristéarates de glycérol 2,0 g
- Triglycérides d'acides gras en C₈-C₁₂ (Miglyol 812) 30,0 g
- Polydiméthyl siloxane 1,5 g
- Alcool cétylique 1,5 g
- Eau distillée qsp 100 g

Cette émulsion est préparée selon les techniques classiques en dissolvant les filtres dans la phase grasse contenant les émulsionnants, en chauffant cette phase grasse vers 80-85°C et en ajoutant, sous vive agitation, l'eau préalablement chauffée vers 80°C.

### EXEMPLE 2

### Huile solaire

On mélange les ingrédients suivants en chauffant éventuellement à 40-45°C pour homogénéiser :
- β,β-diphénylacrylate de 2-éthylhexyle 6,0 g
- 4-tert-butyl-4'-méthoxydibenzoylméthane (PARSOL 1789) 3,0 g
- Myristate d'isopropyle qsp 91,0 g

### EXEMPLE 3

### Huile solaire

On prépare de la même façon que dans l'exemple 2 l'huile solaire suivante :
- β,β-di (4'-méthoxyphényl) acrylate d'éthyle 6,0 g
- 4-tert-butyl-4'-méthoxy dibenzoylméthane (PARSOL 1789) 3,0 g
- Myristate d'isopropyle qsp 91,0 g

### EXEMPLE 4

### Emulsion non ionique

- β,β-diphénylacrylate de 2-éthylhexyle 6 0 g
- 4-tert.-butyl-4'-méthoxy dibenzoylméthane 1,5 g
- Mélange d'alcool cétylstéarylique et d'alcool cétylstéarylique oxyéthyléné à 33 moles d'oxyde d'éthylène 7,0 g
- Mélange de mono-, di- et tristéarates de glycérol 2,0 g
- Triglycérides d'acides gras en C₈-C₁₂ (Miglyol 812) 30,0 g
- Polydiméthyl siloxane 1,5 g
- Alcool cétylique 1,5 g
- Eau distillée qsp 100 g

Cette émulsion est préparée comme dans l'exemple 1.

### EXEMPLE 5

### Emulsion non ionique eau-dans-l'huile

- β,β-di (4'-méthoxyphényl) acrylate d'éthyle 3,0 g
- 4-tert.-butyl-4'-méthoxy dibenzoylméthane 2,0 g
- Mélange d'alcool cétylstéarylique et d'alcool cétylstéarylique oxyéthyléné à 33 moles d'oxyde d'éthylène 7,0 g
- Mélange de mono-, di- et tristéarates de glycérol 2,0 g
- Triglycérides d'acides gras en C₈-C₁₂ (Mygliol 812) 30,0 g
- Polydiméthyl siloxane 1,5 g
- Alcool cétylique 1,5 g
- Eau distillée qsp 100 g

Cette émulsion est préparée comme dans l'exemple 1.

### EXEMPLE 6

### Emulsion huile-dans-l'eau

- α-cyano-β,β-diphénylacrylate de 2-éthylhexyle (UVINUL N 539) 5,0 g
- 4-isopropyldibenzoylméthane (EUSOLEX 8020) 3,0 g
- Mélange d'alcool cétylstéarylique et d'alcool cétylstéarylique oxyéthyléné à 33 moles d'oxyde d'éthylène vendu sous la dénomination "SINNOWAX AO" par la Société HENKEL 7,0 g
- Mélange de mono- et distéarate de glycérol non autoémulsionnable 2,0 g
- Alcool cétylique 1,5 g
- Huile de silicone 1,5 g
- Huile de vaseline 16,0 g
- Glycérine 20,0 g
- Parfum, conservateur qs
- Eau qsp 100 g

On réalise l'émulsion en additionnant la phase grasse contenant les filtres et les émulsionnants aux environs de 80°C à la phase aqueuse portée à la même température et sous agitation rapide.

### EXEMPLE 7

### Emulsion huile-dans-l'eau

- α-cyano-β,β-diphénylacrylate d'éthyle (UVINUL N 35) 2,0 g
- 4-tert.-butyl-4'-méthoxydibenzoylmétane (PARSOL 1789) 1,0 g
- Mélange d'alcool cétylstéarylique et d'alcool cétylstéarylique oxyéthyléné à 33 moles d'oxyde d'éthylène vendu sous la dénomination "SINNOWAX AO" par la Société HENKEL 7,0 g
- Mélange de mono-et distéarate de glycérol non autoémulsionnable 2,0 g
- Alcool cétylique 1,5 g
- Huile de silicone 1,5 g
- Huile de vaseline 20,0 g
- Glycérine 20,0 g
- Parfum, conservateur qs
- Eau qsp 100 g

Cette émulsion est préparée comme dans l'exemple 6.

### EXEMPLE 8

### Emulsion huile dans l'eau

- α-cyano-β,β-diphénylacrylate de 2-éthylhexyle (UVINUL N 539) 8,0 g
- 4-tert.-butyl-4'-méthoxydibenzoylmétane (PARSOL 1789) 1,0 g
- Mélange d'alcool cétylstéarylique et d'alcool cétylstéarylique oxyéthyléné à 33 moles d'oxyde d'éthylène vendu sous la dénomination "SINNOWAX AO" par la Société HENKEL 7,0 g
- Mélange de mono et distéarate de glycérol non autoémulsionnable 2,0 g
- Alcool cétylique 1,5 g
- Huile de silicone 1,5 g
- Huile de vaseline 15,0 g
- Acide benzène 1,4- di(3-méthylidène 10-campho- sulfonique) 1,0 g MA
- Glycérine 20,0 g
- Parfum, conservateur qs
- Eau qsp 100 g

On prépare cette émulsion comme dans l'exemple 6, le filtre hydroxoluble étant dissous dans la phase aqueuse.

## Revendications

1. Composition cosmétique filtrante photostable pour la protection de l'épiderme humain contre les rayons ultraviolets de longueurs d'onde comprises entre 280 et 380 nm, caractérisée par le fait qu'elle comprend, dans un support cosmétiquement acceptable contenant au moins une phase grasse, 1 à 5% en poids de dérivé du dibenzoylméthane et au moins 1% en poids d'un β,β-diphénylacrylate ou α-cyano-β,β-diphénylacrylate d'alkyle de formule : 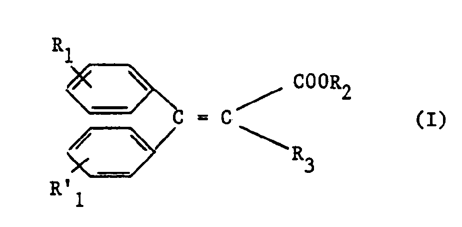 dans laquelle
R₁ et R'₁, identiques ou différents, représentent un atome d'hydrogène, un radical alcoxy en C₁-C₈ à chaîne droite ou ramifiée ou un radical alkyle en C₁-C₄ à chaîne droite ou ramifiée, R₁ et R'₁ étant en position para ou méta; R₂ représente un radical alkyle en C₁-C₁₂ à chaîne droite ou ramifiée et
R₃ représente un atome d'hydrogène ou le radical -CN, le rapport molaire du composé de formule (I) au dérivé de dibenzoylméthane étant supérieur ou égal à 0,8.

2. Composition cosmétique filtrante selon la revendication 1, caractérisée par le fait que le rapport molaire du composé de formule (I) au dérivé de dibenzoylméthane est inférieur ou égal à 8.

3. Composition cosmétique filtrante selon la revendication 1 ou 2, caractérisée par le fait que le dérivé de dibenzoylméthane est choisi parmi les composés suivants : 2-méthyldibenzoylméthane, 4-méthyldibenzoylméthane, 4-isopropyldibenzoylméthane, 4-tert.-butyl- dibenzoylméthane, 2,4-diméthyldibenzoylméthane, 2,5-diméthyldibenzoylméthane, 4,4'-diisopropyldibenzoylméthane, 1-tert.-butyl-4'- méthoxydibenzoylméthane, 2-méthyl-5-isopropyl-4'-méthoxydibenzoyl- méthane, 2-méthyl-5-tert.-butyl-4'-méthoxydibenzoylméthane, 2,4-di- méthyl-4 '-méthoxydibenzoylméthane et 2,6-diméthyl-4-tert.-butyl-4'- méthoxydibenzoylméthane.

4. Composition cosmétique filtrante selon la revendication 3, caractérisée par le fait que le dérivé de dibenzoylméthane est choisi parmi le 4-tert.-butyl-4'-méthoxydibenzoylméthane et le 4-isopropyldibenzoylméthane.

5. Composition cosmétique filtrante selon l'une quelconque des revendications 1 à 4, caractérisée par le fait que le β,β-diphénylacrylate ou α-cyano-β,β-diphénylacrylate d'alkyle est choisi parmi les composés suivants : α-cyano-β,β-diphénylacrylate de 2-éthylhexyle, α-cyano-β,β-diphénylacrylate d'éthyle, β,β-diphénylacrylate de 2-éthylhexyle, et β,β-di(4'-méthoxyphényl)acrylate d'éthyle.

6. Composition cosmétique filtrante selon l'une quelconque des revendications 1 à 5, caractérisée par le fait que le rapport molaire du composé de formule (I) au dérivé de dibenzoylméthane est compris entre 1,2 et 8.

7. Composition cosmétique filtrante selon l'une quelconque des revendications 1 à 6, caractérisée par le fait qu'elle se présente sous forme de lotion huileuse ou oléoalcoolique, sous forme de gel gras ou oléoalcoolique, de bâtonnet solide, d'émulsion, de dispersion vésiculaire de lipides amphiphiles ioniques ou non-ioniques ou est conditionnée en aérosol.

8. Composition cosmétique filtrante selon l'une quelconque des revendications 1 à 7, caractérisée par le fait qu'elle contient en outre des adjuvants cosmétiques choisis parmi les épaississants, adoucissants, humectants, tensio-actifs, conservateurs, anti-mousses, parfums, huiles, cires, mono-alcools et polyols inférieurs, propulseurs, colorants et pigments.

9. Composition cosmétique filtrante selon l'une quelconque des revendications 1 à 8, caractérisée par le fait qu'elle constitue une émulsion sous forme de crème ou de lait comprenant à titre de support cosmétique, des alcools gras, des esters d'acides gras et notamment des triglycérides d'acides gras, des acides gras, de la lanoline, des huiles et des cires naturelles ou synthétiques et des émulsionnants, en présence d'eau.

10. Composition cosmétique filtrante sous forme d'émulsion ou de dispersion vésiculaire selon l'une quelconque des revendications 1 à 9, caractérisée par le fait qu'elle contient en outre des filtres UV hydrosolubles.

11. Composition cosmétique filtrante selon l'une quelconque des revendications 1 à 8, caractérisée par le fait qu'elle constitue une lotion huileuse comprenant à titre de support cosmétique, des esters d'acides gras, des huiles et des cires naturelles ou synthétiques.

12. Composition cosmétique filtrante selon l'une quelconque des revendications 1 à 8, caractérisée par le fait qu'elle constitue une lotion oléoalcoolique comprenant à titre de support cosmétique des huiles, des cires ou des esters d'acides gras et notamment des triglycérides d'acides gras et des alcools, glycols et/ou polyols inférieurs.

13. Composition cosmétique filtrante selon l'une quelconque des revendications 1 à 8, caractérisée par le fait qu'elle constitue un gel oléoalcoolique comprenant à titre de support cosmétique une huile ou une cire naturelle ou synthétique, un alcool ou polyol inférieur et un épaississant.

14. Procédé de traitement cosmétique de l'épiderme humain en vue de sa protection contre le rayonnement UV de longueurs d'onde comprises entre 280 et 380 nm, caractérisé par le fait qu'il consiste à appliquer sur la peau une quantité efficace d'un composition cosmétique filtrante telle que définie dans l'une quelconque des revendications 1 à 13.

15. Procédé de stabilisation de dérivés du dibenzoylméthane vis-à-vis du rayonnement UV de longueurs d'onde comprises entre 280 et 380 nm, caractérisé par le fait qu'on ajoute au moins 1% en poids de composé de formule (I) défini dans la revendication 1, à 1 à 5% en poids de dérivé de dibenzoylméthane, le rapport molaire du composé de formule (I) au dérivé de dibenzoylméthane étant égal ou supérieur à 0,8.

16. Procédé selon la revendication 15, caractérisé par le fait que le rapport molaire du composé de formule (I) au dérivé de dibenzoylméthane est inférieur ou égal à 8.

17. Procédé selon la revendication 15 ou 16, caractérisé par le fait que le rapport molaire du composé de formule (I) au dérivé de dibenzoylméthane est compris entre 1,2 et 8.

18. Procédé de stabilisation de dérivés du dibenzoylméthane vis-à-vis du rayonnement UV de longueurs d'onde comprises entre 280 et 380 nm selon l'une quelconque des revendications 15 à 17, caractérisé par le fait que le dérivé du dibenzoylméthane est le 4-tert.-butyl-4'-méthoxydibenzoylméthane ou le 4-isopropyldibenzoylméthane et le composé de formule (I) est choisi parmi 1'α-cyano-β,β diphénylacrylate de 2-éthylhexyle, l'α-cyano-β,β-diphényl- acrylate d'éthyle, le β,β-diphénylacrylate de 2-éthylhexyle et le β,β-di(4'-méthoxy phényl) acrylate d'éthyle.

## Claims

1. Photostable cosmetic screening composition for protection of the human epidermis against ultraviolet rays of wavelengths between 280 and 380 nm, characterised in that it comprises, in a cosmetically acceptable vehicle containing at least one fatty phase, 1 to 5 % by weight of dibenzoylmethane derivative and at least 1 % by weight of an alkyl β,β-diphenylacrylate or α-cyano-β,β-diphenylacrylate of formula: 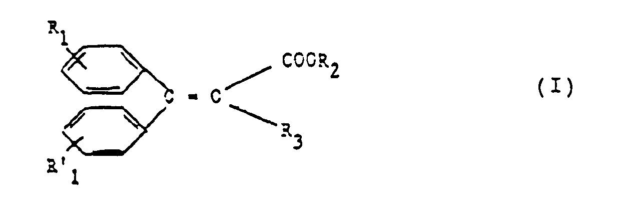 in which
R₁ and R'₁, which may be identical or different, represent a hydrogen atom, a straight- or branched-chain C₁-C₈ alkoxy radical or a straight- or branched-chain C₁-C₄ alkyl radical, R₁ and R'₁ being in the para or meta position; R₂ represents a straight- or branched-chain C₁-C₁₂ alkyl radical; and
R₃ represents a hydrogen atom or a -CN radical, the mole ratio of the compound of formula (I) to the dibenzoylmethane derivative being not less than 0.8.

2. Cosmetic screening composition according to Claim 1, characterised in that the mole ratio of the compound of formula (I) to the dibenzoylmethane derivative is not more than 8.

3. Cosmetic screening composition according to Claim 1 or 2, characterised in that the dibenzoylmethane derivative is selected from the following compounds: 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4-tert-butyl-4'-methoxydibenzoylmethane, 2-methyl-5-isopropyl-4'-methoxydibenzoylmethane, 2-methy-5-tert-butyl-4'-methoxydibenzoylmethane, 2,4-dimethyl-4 '-methoxydibenzoylmethane and 2,6-dimethyl-4-tert-butyl-4'-methoxydibenzoylmethane.

4. Cosmetic screening composition according to Claim 3, characterised in that the dibenzoylmethane derivative is selected from 4-tert-butyl-4'-methoxydibenzoylmethane and 4-isopropyldibenzoylmethane.

5. Cosmetic screening composition according to any one of Claims 1 to 4, characterised in that the alkyl β,β-diphenylacrylate or α-cyeno-β,β-diphenylecrylate is selected from the following compounds: 2-ethylhexyl α-cyano-β,β-diphenylacrylate, ethyl α-cyano-β,β-diphenylacrylate, 2-ethylhexyl β,β-diphenylacrylate and ethyl β,β-bis(4-methoxyphenyl)acrylate.

6. Cosmetic screening composition according to any one of Claims 1 to 5, characterised in that the mole ratio of the compound of formula (I) to the dibenzoylmethane derivative is between 1.2 and 8.

7. Cosmetic screening composition according to any one of Claims 1 to 6, characterised in that it takes the form of an oily or oleoalcoholic lotion or the form of a fatty or oleoalcoholic gel, of a solid stick, of an emulsion or of a vesicular dispersion of ionic or nonionic amphiphilic lipids, or is packaged as an aerosol.

8. Cosmetic screening composition according to any one of Claims 1 to 7, characterised in that it contains, in addition, cosmetic adjuvants selected from thickeners, emollients, humectants, surfactants, preservatives, antifoams, fragrances, oils, waxes, lower polyols and monohydric alcohols, propellants, colourings and pigments.

9. Cosmetic screening composition according to any one of Claims 1 to 8, characterised in that it constitutes an emulsion in the form of a cream or milk comprising, as a cosmetic vehicle, fatty alcohols, fatty acid esters, and in particular fatty acid triglycerides, fatty acids, lanolin, natural or synthetic oils and waxes and emulsifiers, in the presence of water.

10. Cosmetic screening composition in the form of an emulsion or of a vesicular dispersion according to any one of Claims 1 to 9, characterised in that it contains, in addition, water-soluble UV screening gents.

11. Cosmetic screening composition according to any one of Claims 1 to 8, characterised in that it constitutes an oily lotion comprising, as a cosmetic vehicle, fatty acid esters, oils and natural or synthetic waxes.

12. Cosmetic screening composition according to any one of claims 1 to 8, characterised in that it constitutes an olecalcoholic lotion comprising, as a cosmetic vehicle, oils, waxes or fatty acid esters, and in particular fatty acid triglycerides, and lower polyols, alcohols and/or glycols.

13. Cosmetic screening composition according to any one of Claims 1 to 8, characterised in that it constitutes an olecalcoholic gel comprising, as a cosmetic vehicle, a natural or synthetic oil or wax, a lower polyol or alcohol and a thickener.

14. Process for a cosmetic treatment of the human epidermis in view of its protection against UV radiation of wavelengths between 280 and 380 nm, characterised in that it consists in applying to the skin an effective quantity of a cosmetic screening composition as defined in any one of Claims 1 to 13.

15. Process for stabilising dibenzoylmethane derivatives with respect to UV radiation of wavelengths between 280 and 380 nm, characterised in that at least 1 % by weight of compound of formula (I) defined in Claim 1 is added to 1 to 5 % by weight of dibenzoylmethane derivative, the mole ratio of the compound of formula (I) to the dibenzoylmethane derivative being not less than 0.8.

16. Process according to Claim 15, characterised in that the mole ratio of the compound of formula (I) to the dibenzoylmethane derivative is not more than 8.

17. Process according to Claim 15 or 16, charac- terised in that the mole ratio of the compound of formula (I) to the dibenzoylmethane derivative is between 1.2 and 8.

18. Process for stabilising dibenzoylmethane derivatives with respect to UV radiation of wavelengths between 280 and 380 nm according to any one of Claims 15 to 17, characterised in that the dibenzoylmethane derivative is 4-tert-butyl-4'-methoxydibenzoylmethane of 4-isopropyl- dibenzoylmethane and the compound of formula (I) is selected from 2-ethylhexyl α-cyano-β,β-diphenylacrylate, ethyl α-cyano-β,β-diphenylacrylate, 2-ethylhexyl β,β- diphenylacrylate and ethyl β,β-bis(4-methoxyphenyl)- acrylate.

## Patentansprüche

1. Lichtbeständige filternde kosmetische Zusammensetzung zum Schutz der menschlichen Epidermis vor ultravioletten Strahlen der Wellenlängen zwischen 280 und 380 nm, **dadurch** **gekennzeichnet,** daß sie in einem kosmetisch akzeptablen Träger, der mindestens eine fette Phase umfaßt, 1 bis 5 Gew.-% eines Derivats von Dibenzoylmethan und mindestens 1 Gew.-% β,β-Diphenylacrylsäurealkylester oder α-Cyano-β,β-diphenylacrylsäurealkylester der Formel 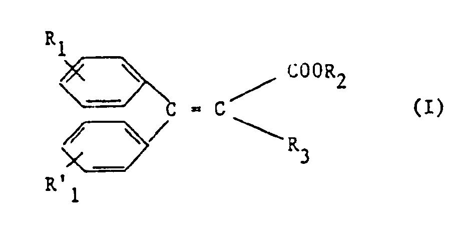 enthält, in der R₁ und R₁' gleich oder verschieden sind und jeweils ein Wasserstoffatom, eine geradkettige oder verzweigte Alkoxygruppe mit 1 bis 8 Kohlenstoffatomen oder eine geradkettige oder verzweigte Alkylgruppe mit 1 bis 4 Kohlenstoffatomen darstellen; R₁ und R₁' sich in para- oder meta-Stellung befinden; R₂ eine geradkettige oder verzweigte Alkylgruppe mit 1 bis 12 Kohlenstoffatomen darstellt, und R₃ ein Wasserstoffatom oder die CN-Gruppe darstellt; und daß das molare Verhältnis der Verbindung der Formel (I) zum Derivat von Dibenzoylmethan gleich 0,8 oder höher ist.

2. Filternde kosmetische Zusammensetzung nach Anspruch 1, **dadurch gekennzeichnet,** daß das molare Verhältnis der Verbindung der Formel (I) zum Derivat von Dibenzoylmethan gleich 8 oder niedriger ist.

3. Filternde kosmetische Zusammensetzung nach Anspruch 1 oder 2, **dadurch gekennzeichnet,** daß das Derivat von Dibenzoylmethan unter den folgenden Verbindungen ausgewählt ist:
2-Methyldibenzoylmethan, 4-Methyldibenzoylmethan, 4-Isopropylidendibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4-tert.-Butyl-4'-methoxydibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan und 2,6-Dimethyl-4-tert.-butyl-4'-methoxydibenzoylmethan.

4. Filternde kosmetische Zusammensetzung nach Anspruch 3, **dadurch gekennzeichnet,** daß das Derivat unter 4-tert.-Butyl-4'-methoxydibenzoylmethan und 4-Isopropyldibenzoylmethan ausgewählt ist.

5. Filternde kosmetische Zusammensetzung nach einem der Ansprüche 1 bis 4, **dadurch gekennzeichnet,** daß der β,β- Diphenylacrylsäurealkyester oder der α-Cyano-β,β-diphenylacrylsäurealkylester unter den folgenden Verbindungen ausgewählt ist:
α-Cyano-β,β-diphenylacrylsäure-(2-ethylhexyl)ester; α-Cyano-β,β-diphenylacrylsäureethylester, β,β-Diphenylacrylsäure-(2-ethylhexyl-)ester und β,β-Di(4'-methoxyphenyl)-acrylsäureethylester.

6. Filternde kosmetische Zusammensetzung nach einem der Ansprüche 1 bis 5, **dadurch gekennzeichnet,** daß das molare Verhältnis der Verbindung der Formel (I) zum Derivat von Dibenzoylmethan zwischen 1,2 und 8 liegt.

7. Filternde kosmetische Zusammensetzung nach einem der Ansprüche 1 bis 6, **dadurch gekennzeichnet,** daß sie als ölige oder ölig-alkoholische Lotion, als fetthaltiges oder öligalkoholisches Gel, als festes Stäbchen, als Emulsion, als vesikuläre Dispersion von amphiphilen ionischen oder nichtionischen Lipiden vorliegt oder daß sie als Spray hergestellt ist.

8. Filternde kosmetische Zusammensetzung nach einem der Ansprüche 1 bis 7, **dadurch gekennzeichnet,** daß sie außerdem kosmetische Zusatzstoffe enthält, die unter Verdickungsmitteln, Weichmachern, feuchtigkeitsspendenden Mitteln, oberflächenaktiven Mitteln, Konservierungsstoffen, Entschäumern, Parfums, Ölen, Wachsen, Monoalkoholen und niedrigen Polyalkoholen, Treibmitteln, Farbstoffen und Pigmenten ausgewählt sind.

9. Filternde kosmetische Zusammensetzung nach einem der Ansprüche 1 bis 8, **dadurch gekennzeichnet,** daß sie eine Emulsion in Form einer Creme oder einer Milch darstellt, die als kosmetischen Träger Fettalkohole, Fettsäureester- und insbesondere Triglyceride von Fettsäuren, Fettsäuren, Lanolin, natürliche oder synthetische Öle und Wachse sowie Emulgatoren in Gegenwart von Wasser enthält.

10. Filternde kosmetische Zusammensetzung als Emulsion oder vesikuläre Dispersion nach einem der Ansprüche 1 bis 9, **dadurch gekennzeichnet,** daß sie außerdem wasserlösliche UV-Filter enthält.

11. Filternde kosmetische Zusammensetzung nach einem der Ansprüche 1 bis 8, **dadurch gekennzeichnet,** daß sie eine ölige Lotion darstellt, die als kosmetischen Träger Fettsäureester, natürliche oder synthetische Öle oder Wachse enthält.

12. Filternde kosmetische Zusammensetzung nach einem der Ansprüche 1 bis 8, **dadurch gekennzeichnet,** daß sie eine öligalkoholische Lotion ist, die als kosmetischen Träger Öle, Wachse oder Fettsäureester und insbesondere Triglyceride von Fettsäuren sowie Alkohole, Glykole und/oder niedrigere Polylakohole enthält.

13. Filternde kosmetische Zusammensetzung nach einem der Ansprüche 1 bis 8, **dadurch gekennzeichnet,** daß sie ein öligalkoholisches Gel darstellt, das als kosmetischen Träger ein natürliches oder synthetisches Öl oder Wachs, einen Alkhohol oder einen niedrigeren Polyalkohol und ein Verdickungsmittel enthält.

14. Verfahren zur kosemtischen Behandlung der menschlichen Epidermis zu ihrem Schutz vor UV-Strahlen der Wellenlängen zwischen 280 und 380 nm, **dadurch gekennzeichnet,** daß es darin besteht, eine wirksame Menge einer filternden kosmetischen Zusammensetzung, wie sie in einem der Ansprüche 1 bis 13 definiert ist, auf die Haut aufzutragen.

15. Verfahren zur Stabilisierung von Dibenzoylmethan-Derivaten gegenüber UV-Strahlung der Wellenlängen zwischen 280 und 380 nm, **dadurch gekennzeichnet,** daß man mindestens 1 Gew.-% der Verbindung der Formel (I), die in Anspruch 1 definiert ist, 1 bis 5 Gew.-% des Derivats von Dibenzoylmethan zusetzt, wobei das molare Verhältnis der Verbindung der Formel (I) zu dem Derivat von Dibenzoylmethan gleich 0,8 oder mehr ist.

16. Verfahren nach Anspruch 15, **dadurch gekennzeichnet,** daß das molare Verhältnis der Verbindung der Formel (I) zu dem Derivat von Dibenzoylmethan gleich 0,8 oder weniger ist.

17. Verfahren nach Anspruch 15 oder 16, **dadurch** **gekennzeichnet,** daß das molare Verhältnis der Verbindung der Formel (I) zu dem Derivat von Dibenzoylmethan zwischen 1,2 und 8 ist.

18. Verfahren zur Stabilisierung von Dibenzyol-Derivaten gegenüber UV-Strahlung der Wellenlängen zwischen 280 und 380 nm nach einem der Ansprüche 15 bis 17, **dadurch** **gekennzeichnet,** daß das Dibenzoylmethan-Derivat 4-tert.-Butyl-4'-methoxydibenzoylmethan oder 4-Isopropyldibenzoylmethan ist, und die Verbindung der Formel (I) unter α-Cyano-β,β-diphenylacrylsäure-(2-ethylhexyl)ester, α-Cyano-β,β-diphenylacrylsäureethylester; β,β-Diphenylacrylsäure-(2-ethylhexyl)ester und β,β-Di(4'-methoxyphenyl)-acryl-säureethylester ausgewählt ist.
